## Europäisches Patentamt
## European Patent Office
## Office européen des brevets

(19)

(11) Veröffentlichungsnummer: **0 052 867**
**A1**

(12)

# EUROPÄISCHE PATENTANMELDUNG

(21) Anmeldenummer: **81109783.1**

(22) Anmeldetag: **19.11.81**

(51) Int. Cl.³: **C 12 P 1/04**
**C 12 N 1/20**
**//(C12P1/04, C12R1/01)**

(30) Priorität: **19.11.80 DE 3043642**
**17.11.81 DE 3145621**

(43) Veröffentlichungstag der Anmeldung:
**02.06.82 Patentblatt 82/22**

(84) Benannte Vertragsstaaten:
**BE IT**

(71) Anmelder: **Gesellschaft für Biotechnologische Forschung mbH (GBF)**
**Mascheroder Weg 1**
**D-3300 Braunschweig-Stöckheim(DE)**

(72) Erfinder: **Augustiniak, Herrmann**
**In den Lindendöhren 19**
**D-3340 Wolfenbüttel(DE)**

(72) Erfinder: **Gerth, Klaus**
**Am Butterbusch 17**
**D-3300 Braunschweig(DE)**

(72) Erfinder: **Höfle, Gerhard**
**Am Windmühlenberg 2**
**D-3300 Braunschweig(DE)**

(72) Erfinder: **Irschik, Herbert**
**Alter Weg 5**
**D-3340 Wolfenbüttel(DE)**

(72) Erfinder: **Kemmer, Torsten**
**Schützenstrasse 2**
**D-3340 Wolfenbüttel(DE)**

(72) Erfinder: **Kohl, Werner**
**Wolfenbütteler Strasse 46**
**D-3300 Braunschweig(DE)**

(72) Erfinder: **Kunze, Brigitte**
**Wolfenbütteler Strasse 43**
**D-3300 Braunschweig(DE)**

(72) Erfinder: **Reichenbach, Hans**
**Platanenstrasse 35**
**D-3340 Wolfenbüttel(DE)**

(72) Erfinder: **Santoso, Sentot**
**Admonter Ring 51**
**D-6301 Pohlheim 2(DE)**

(72) Erfinder: **Steinmetz, Heinrich**
**Sandgrubenweg 32**
**D-3300 Braunschweig(DE)**

(72) Erfinder: **Trowitzsch, Wolfram**
**Kollwitzstrasse 14**
**D-3300 Braunschweig(DE)**

(74) Vertreter: **Boeters, Hans Dietrich, Dr. et al,**
**Boeters, Bauer & Partner Thomas-Wimmer-Ring 14**
**D-8000 München 22(DE)**

(54) Kulturbrühe, Extrakt, Wirkstoffgemisch, Wirkstoffe sowie Verfahren zu deren Herstellung sowie Mikroorganismen.

(57) Die Erfindung betrifft eine Kulturbrühe von Myxococcus virescens Mx v 48, DSM 1888, DSM 2177, und seiner Mutant Mx-v-48M, einen Extrakt, ein Wirkstoffgemisch und Wirkstoffe mit antibiotischer Wirkung. Charakterisierung einzelner Wirkstoffe aus dem Gemisch von Mx-v-48 M8 : Wirkstoffe A und A-1.

Der die Hauptmenge des Wirkstoffgemisches bildende Wirkstoff A kann aus dem Gemisch mittels Chromatographie an reversed-phase Materialien isoliert werden. A besteht aus zwei isomeren Substanzen, aus denen A-1 mittels fraktionierter Kristallisation erhalten wird (Lösungsmittelgemisch: Hexan/Dichlormethan)

Massenspektrum von A und A-1 (70 eV, 230°C, MS 9) $C_{35}H_{61}NO_8$ (Hochauflösung) gef.: 623.4395, ber.: 623,4397

Das Molekül-Ion verliert Methanol und geht über in $C_{34}H_{57}NO_7$ gef.: 591,4136
ber.: 591,4138
Dieses Fragment-Ion verliert $C_5H_8O_2$ und geht über in $C_{29}H_{49}NO_5$ gef.: 491,3610
ber.: 491,3610
Optische Drehung von A-1 : $\alpha_D = + 27.3°$, c=1 (Methanol)

./...

Wirkstoff *B*

*Massenspektrum*

Durch Hochauflösung wird die Summenformel zu $C_{35}H_{59}NO_8$ ermittelt.

Gefunden wurde: 621.4257, für obige Summenformel berechnet: 621.4240.

Wirkstoffe *C* und *D*

*Massenspektrum*

Die Summenformel $C_{35}H_{63}NO_7$ wird durch Hochauflösung ermittelt.

Gef.: 609,4599, für die Summenformel berechnet: 609,4604.

*Optische Drehungen*

*C*: $\alpha_D = +22.2°$ (c=0.3 ,$CHCl_3$),

*D*: $\alpha_D = +6.3°$ (c=0.25,$CHCl_3$)

*Massenspektrum*

Durch Hochauflösung wurde die Summenformel $C_{32}H_{55}NO_8$ bestimmt.

Gef.: 581.39200, für die Formel ber.: 581.39274.

**BOETERß, BAUER & PARTNER**
PATENTANWÄLTE
EUROPEAN PATENT ATTORNEYS
THOMAS-WIMMER-RING 14
D-8000 M Ü N C H E N 22

PAs BOETERS, BAUER & PARTNER
THOMAS-WIMMER-RING 14, D-8000 MÜNCHEN 22

DIPL-CHEM. DR. HANS D. BOETERS
DIPL.-ING. ROBERT BAUER
MÜNCHEN

DIPL.-ING. VINCENZ v. RAFFAY
DIPL.-CHEM. DR. THOMAS FLECK
HAMBURG

TELEFON: (089) 22 78 87
TELEX: 5 24 878 rrm
TELEGRAMME: PROVENTION, MÜNCHEN

0052867

**Kulturbrühe, Extrakt, Wirkstoffgemisch, Wirkstoffe
sowie Verfahren zu deren Herstellung** sowie Mikroorganismen

Die Erfindung betrifft eine neue Kulturbrühe, einen neuen Extrakt, ein neues Wirkstoffgemisch und neue Wirkstoffe, die unter Verwendung eines neuen Mikroorganismus gewonnen werden. Ferner betrifft die Erfindung Verfahren zur Herstellung der Kulturbrühe, des Extraktes, des Wirkstoffgemisches und der Wirkstoffe.

Herkunft und Hinterlegung des Mikroorganismus
Bei dem neuen Mikroorganismus handelt es sich um einen Stamm des Bakteriums Myxococcus virescens der Klasse Flexibacteriae, der Ordnung Myxobacterales und der Familie Myxococcaceae. Der Stamm Mx v 48 wurde im November 1977 aus einer Probe von Kaninchenmist, der im April 1977 im Osten Teneriffas, Spanien, bei Punta del Hidalgo, gesammelt worden war, isoliert. Hinterlegungen bei der Deutschen Sammlung von Mikroorganismen/Göttingen:

| Bezeichnung | Hinterlegungs-Nr. | Hinterlegungstag |
|---|---|---|
| Mx v 48 | DSM 1888 | 19. 8. 1980 |
| Mx v 48-M(utante) 8 | DSM 2177 | |

Beschreibung des Stamms

Die vegetativen Zellen sind zylinderförmige Stäbchen von etwa 4 bis 6 $\mu$m Länge und einer Dicke von etwa 0,8 bis 0,9 $\mu$m mit sich verjüngenden Enden ("zigarrenförmig").

Die Myxosporen weisen eine angenäherte Kugelform mit einem Durchmesser von etwa 1,8 bis 2,3 $\mu$m auf und erscheinen im Phasenkontrastmikroskop stark lichtbrechend.

Die Zellen bewegen sich gleitend fort und die Kolonien breiten sich schwarmförmig allmählich auf der Oberfläche einer Kulturplatte aus.

Auf einem peptonhaltigen Kultursubstrat (z.B. cy-Agar) zeigt der Schwarm meist eine kräftige gelbgrüne Färbung.

-cy-Agar: 0,3 % Casitone (Difco) 0,1 % Hefe-Extrakt (Difco)

0,1 % $CaCl_2$ x $2H_2O$

1,5 % Agar

pH 7,2

Auf Agar mit Bäckerhefe (z.B. vy/2-Agar) ist der Stamm meist farblos. Hefezellen werden kräftig lysiert.

-vy/2-Agar: 0,5 % Bäckerhefe (bezogen auf Frischgewicht)

0,1 % $CaCl_2$ x $2H_2O$

0,5 mg/l Cyanocobalamin

1,5 % Agar pH 7,2

Auf vy/2-Agar werden oft weichschleimige, tropfenförmige Fruchtkörper von etwa 200 bis 300 $\mu$m Höhe und etwa 200 bis 300 $\mu$m Durchmesser gebildet, welche eine gelbgrüne oder auch orange bis rote Färbung aufweisen.

In Flüssigmedien wächst der Organismus in homogener Suspension. Beispielsweise werden die Kulturen in Schüttelkolben (z.B. 50 ml in 250 ml-Erlenmeyerkolben) bei 30°C (mit z.B. 160 U/min) geschüttelt. Als Medium läßt sich beispielsweise cas $\ell$.m. verwenden:

1 % Casitone (Difco)

0,1 % $MgSO_4$ x $7H_2O$

pH 6,8

Das Bakterium läßt sich mit folgenden Konservierungsmethoden über längere Zeit am Leben erhalten:
1) Myxosporen in Magermilch getrocknet,
2) vegetative Zellen in cas l.m. bei $-80^{\circ}C$ eingefroren,
3) Fruchtkörper auf Filterpapier getrocknet.

Der Stamm Mx v 48 ist auf die Verwertung von Proteinen und Peptonen eingestellt und baut andere Mikroorganismen (Bakterien und Hefen) ab.

Kulturbrühe

Die Erfindung betrifft eine Kulturbrühe des Stammes Mx v 48 , die durch Kultivieren von Myxococcus virescens Mx v 48 in einem Kohlenstoff , Stickstoff und Mineralsalze enthaltenden wässrigen Nährmedium bei einer Temperatur von etwa 15 bis 40 $^{\circ}C$ erhältlich ist. Vorzugsweise ist die Kulturbrühe durch Kultivieren von Myxococcus virescens Mx v 48 bei Temperaturen von etwa 25 bis 35 $^{\circ}C$, insbesondere bei 30 $^{\circ}C$, erhältlich.

Die Überstände von Flüssigkulturen des Organismus zeigen nach Konzentrieren eine deutliche Aktivität gegenüber gram-negativen Bakterien, insbesondere gegenüber Enterobacteriaceae, jedoch können auch gram-positive Bakterien im Wachstum gehemmt werden.

Das Antibiotikum wird während der exponentiellen Wachstumsphase gebildet. Der optimale Zeitpunkt für den Abbruch der Fermentation kann mittels Messung der optischen Dichte (gemessen bei lambda 623 nm und einer Schichtdicke von 1 cm; $o.D._{623}$) durch ein Eppendorf-Photometer bestimmt werden. Er ist erreicht, wenn die $o.D._{623}$ bei Werten von etwa 0,8 bis 1,2 liegt. Die Kultur wächst anschließend noch linear weiter und erreicht bei Vorlage von 0,5% Pepton eine maximale optische Dichte $o.D._{623\,nm}$ von etwa 2. Zu diesem Zeitpunkt ist das Antibiotikum nicht mehr nachweisbar. Extrakte mit antibiotischer Aktivität, ein Wirkstoffgemisch und Wirkstoffe können aus dem Kulturüberstand gewonnen werden. In den Zellen ist die Aktivität sehr gering.

Im Verlauf einer Stammoptimierung wurde durch mehrfache Mutagenisierungsschritte eine Produktionsmutante Mx v48 M8 erzeugt.

Diese Mutante ist gegenüber ihrem Antibiotikum resistent bis 35 $\mu$g/ml.
Die Generationszeit ist im Vergleich mit dem Wildstamm unverändert, auch
in Gegenwart von 20 $\mu$g Antibiotikum/ml.

Während der Wildstamm das Antibiotikum nur während der logarithmischen
Wachstumsphase produziert und die Aktivität in der stationären Wachstumsphase wieder verschwindet, produziert die Mutante erst gegen Ende der
log Phase mit Beginn einer auftretenden Substratlimitierung. Die Ausbeute
an Antibiotikum liegt bei einer typischen Fermentation bei 2 mg/ml.

Fermentiert wird in folgendem Medium: 0.4 % g/v Corn Steep Pulver
0.1 % g/v Sojabohnenmehl
0.1 % g/v Pepton trypt. Merck (Darmstadt)
0.025 % g/v $MgSO_4 \cdot 7 H_2O$
0.005 % g/v $CaCl_2 \cdot 2 H_2O$
1 mg $CoCl_2 \cdot 6 H_2O$/l

Der Verlauf einer typischen Fermentation ist in den folgenden
4 Diagrammen auf den Seiten 5-8 abgebildet.

ABS.ZEIT( STD:MIN:SEK,BZH.MO/TA/JA)
10/13/81    12:00:00    10/14/81    12:00:00    10/15/81

pH

Temperatur

- TEMP - [ GRD CELS]    - PH -

REL.ZEIT( STD=MIN=SEK)
0=00=00    13=45=00    27=30=00    41=15=00    55=00=00

ABS.ZEIT( STD:MIN:SEK,BZW.MO/TA/JA)

10/13/81    12:00:00    10/14/81    12:00:00    10/15/81

2.00

1.60

1.20

0.80

0.40

0.00

RQ-WERT

REL.ZEIT(STD=MIN=SEK)

0=00=00    13=45=00    27=30=00    41=15=00    55=00=00

0.00

-0.20

ABS.ZEIT( STD:MIN:SEK,BZH.MO/TA/JA)

10/13/81  12:00:00  10/14/81  12:00:00  10/15/81

5.00  21.00

O1

4.00  19.8^

3.00  18.60

[ % ]  [ % ]

2.00  17.40

- CO2 -  - O2 -

1.00  16.20

CO2

0.00  15.00

0:00:00  13:45:00  27:30:00  41:15:00  55:00:00

REL.ZEIT( STD:MIN:SEK)

0052867

Extrakt

Die Erfindung betrifft somit auch einen Extrakt, der erhältlich ist durch Abtrennen der Zellen von Myxococcus virescens Mx-v-48 M8 von einer erfindungsgemäß erhaltenen Kulturbrühe und Extrahieren der abgetrennten Kulturbrühe mit einem organischen Lösungsmittel, wie beispielsweise Essigsäureethylester.

Alternativ wird das Wirkstoffgemisch auch erhalten, wenn Adsorberharz XAD-2 oder XAD-4 (Amberlite) in die Kulturbrühe eingerührt wird, das Harz abfiltriert, mit Wasser gewaschen und anschließend mit Aceton eluiert wird. Das Wirkstoffgemisch läßt sich dabei von weiteren Verunreinigungen befreien, wenn vor der Elution mit Aceton Wasser:Methanol=1:1 zur Eluierung verwendet wird. Dieses Verfahren ist auch als Chromatographie an einer Säule anwendbar.

Wirkstoffgemisch

Die Erfindung betrifft ferner ein Wirkstoffgemisch, das aus dem erfindungsgemäßen Extrakt nach dessen Entwässerung und Einengung durch

(a) Verteilung zwischen einer polaren und einer unpolaren organischen Phase,

(b) Chromatographieren der eingeengten polaren Phase über Sephadex LH-20 und

(c) Chromatographieren an reversed-phase-Kieselgel mit einem polaren organischen Lösungsmittel unter Zusatz von Wasser gewonnen werden kann.

Das Wirkstoffgemisch besteht aus mindestens zwölf Substanzen. Neben der UV-Löschung auf Kieselgel $F_{254}$ können die Wirkstoffe durch Einstellen in eine Jod-Kammer sichtbar gemacht werden.

Nach Ansprühen der Wirkstoffe mit einer Lösung von Hydroxylamin/Eisen-(III)-chlorid bilden sich zunächst je nach Verbindung schwach orange bis rötlich gefärbte Flecken, letztere gehen langsam in eine violette Farbe über.

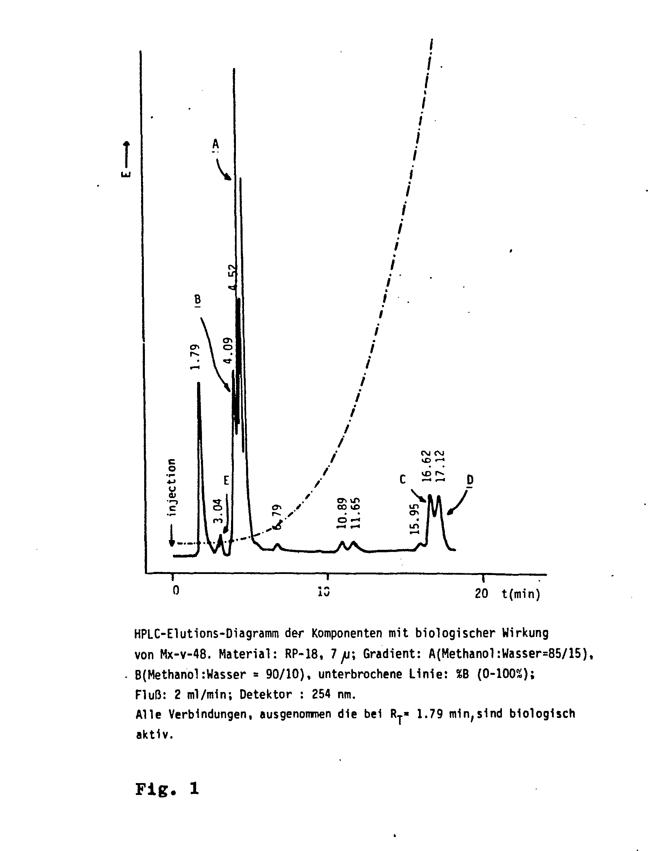

Das HPLC-Elutions-Diagramm des Wirkstoffgemisches an reversed-phase Material ist in der Abbildung 1 dargestellt.

Die im folgenden beschriebene biologische Aktivität gegen E.coli wurde qualitativ bei allen Substanzen, bis auf die mit der Retentionszeit $R_T$= 1.79 nachgewiesen.

Charakterisierung des Wirkstoffgemisches
Die folgende Zusammenstellung zeigt das Wirkungsspektrum der Hauptkomponente A im Plättchendiffusionstest. Die Konzentration des Gemisches betrug 10 /ug/Plättchen. Die Aktivität wurde anhand des

Hemmhofdurchmessers bestimmt. Die Testplatten wurden hergestellt durch Eingießen von 10 ml Agarmedium in Petrischalen
von 9 cm Durchmesser. Der Agar wurde zuvor mit dem jeweiligen Testorganismus beimpft.

| Testorganismus | Hemmhofdurchmesser |
|---|---|
| Escherichia coli | 13 |
| Klebsiella spec. | 12 |
| Proteus mirabilis | 11 |
| Proteus morganii | 9,5 |
| Serratia marcescens | 12 |
| Salmonella typhimurium | 10 |
| Aerobacter aerogenes | 8 |
| Myxococcus virescens Mx v 48 | 10 |
| Streptomyces heteropsis | 8 |
| Caryophanon latum | 18 |
| Azotobacter agilis | 11 |
| Nocardia flava | 14 |
| Alcaligenes eutrophus | 12 |
| Staphylococcus aureus | - |
| Bacillus subtilis | - |
| Bacillus polymyxa | - |
| Bacillus thuringiensis | - |
| Micrococcus lysodeikticus | - |
| Rhizobium meliloti | - |
| Pseudomonas acidovoran | - |
| Pseudomonas aeruginosa | - |
| Arthrobacter rubellus | - |
| Brevibacterium ammoniagenes | - |
| Vibrio extorquens | - |
| Agrobacterium tumefaciens | - |
| Candida albicans | - |
| Nadsonia fulvescens | - |
| Schizosaccharomyces pombe | - |
| Pichia membranaefaciens | - |
| Mucor hiemalis | - |
| Paecilomyces spec. | - |
| Penicillium digitatum | - |

0052867

Die minimale Hemmkonzentration (MHK) wurde im Reihenverdünnungstest ermittelt.

| Testorganismus | MHK ( $\mu$g/ml) |
|---|---|
| Escherichia coli | 1 |
| Myxococcus virescens Mx-v-48 | 0.5 |
| Staphylococcus aureus | 25 |
| Candida albicans | 100 |

In Gegenwart von 10% Rinderserumalbumin erhöht sich die MHK gegenüber E.coli durch Bindung des Wirkstoffes an Albumin auf 3 $\mu$g/ml.

Die Protein- und DNA-Synthese wird bei Escherichia coli in Gegenwart von 4 $\mu$g des erfindungsgemäßen Antibiotikums innerhalb einer Stunde nur wenig gehemmt. Die RNS-Synthese wird gegenüber einer Kontrollkultur innerhalb einer Stunde um 40 % gehemmt.

Charakterisierung einzelner Wirkstoffe aus dem Gemisch von Mx-v-48 M8 Wirkstoffe A und A-1

Der die Hauptmenge des Wirkstoffgemisches bildende Wirkstoff A kann aus dem Gemisch mittels Chromatographie an reversed-phase Materialien isoliert werden. A besteht aus zwei isomeren Substanzen, aus denen A-1 mittels fraktionierter Kristallisation erhalten wird (Lösungsmittelgemisch: Hexan/Dichlormethan)

Chromatographie-Verhalten auf Kieselgel von A und A-1

| Laufmittel-System | $R_f$-Wert |
|---|---|
| Essigsäureäthylester | 0.1 |
| Dichlormethan/Methanol (15/1) | 0.43 |
| Hexan/Isopropanol (4/1) | 0.17 |
| Hexan/Aceton (1/1) | 0.14 |

Massenspektrum von $\underline{A}$ und $\underline{A-1}$ (70 eV, 230°C, MS 9)

$C_{35}H_{61}NO_8$ (Hochauflösung) gef.: 623.4395, ber.: 623,4397

Das Molekül-Ion verliert Methanol und geht über in

$C_{34}H_{57}NO_7$      gef.: 591, 4136

                ber.: 591, 4138

Dieses Fragment-Ion verliert $C_5H_8O_2$ und geht über in

$C_{29}H_{49}NO_5$      gef.: 491, 3610

                ber.: 491, 3610

<u>Optische Drehung von A-1</u> : $\alpha_D = + 27.3°$, c=1 (Methanol) UV-Spektrum (Methanol):

(Abbildung 2)      $\text{lambda}_{max} = 238$ nm (epsilon = 21 800) Schulter bei 280 nm (epsilon = 300)

IR-Spektrum ($CHCl_3$):

(Abbildung 3)      $\bar{\nu} = 3450$, 3000, 2970, 2930, 2875, 1740, 1707, 1665, 1525, 1460, 1400, 1380, 1360, 1240, 1090, 970 und 908 $cm^{-1}$.

$^1$H-NMR-Spektrum ($CDCl_3$)

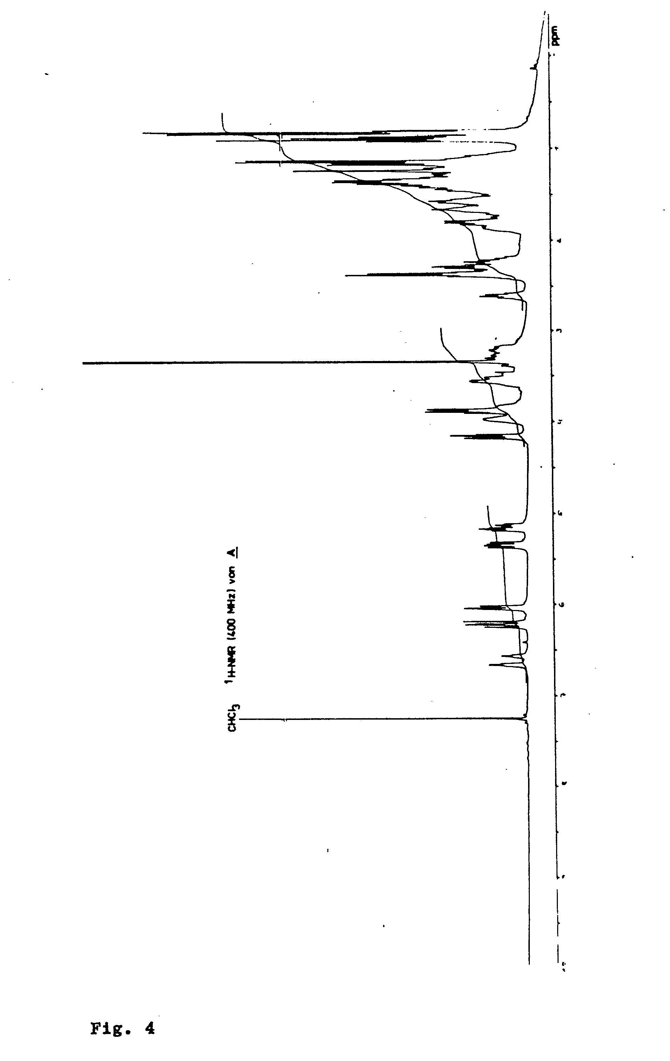

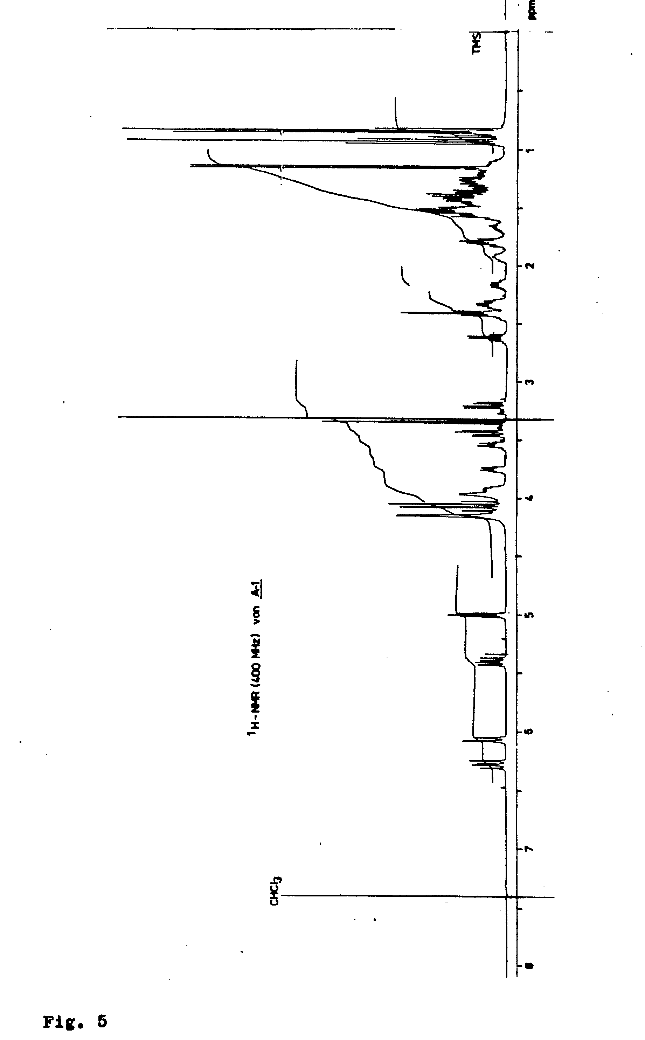

von $\underline{A}$ , siehe Abbildung 4, von $\underline{A-1}$, siehe Abbildung 5

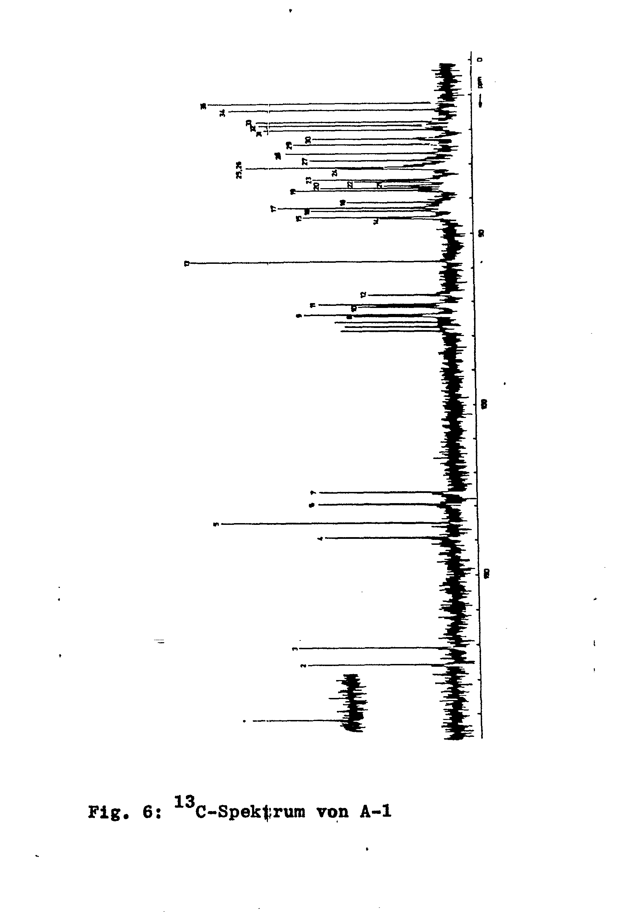

$^{13}$C-NMR-Spektrum ($CDCl_3$) (von $\underline{A-1}$, siehe Abbildung 6 )

Die chemischen Verschiebungen (delta, ppm) der entsprechend numerierten Kohlenstoffatome sind nachfolgend zusammengestellt:

Tabelle 2

| | | | | |
|---|---|---|---|---|
| 1 | - | 212,2 | 11 - | 70,5 |
| 2 | - | 176,1 | 12 - | 67,7 |
| 3 | - | 171,1 | 13 - | 57,7 |
| 4 | - | 139,2 | 14 - | 45,3 |
| 5 | - | 134,9 | 15 - | 44,9 |
| 6 | - | 129,3 | 16 - | 43,0 |
| 7 | - | 125,7 | 17 - | 42,1 |
| 8 | - | 74,0 | 18 - | 40,6 |
| 9 | - | 73,5 | | |
| 10 | - | 71,2 | | |

| | | | | | |
|---|---|---|---|---|---|
| 19 | — | 37,0 | 28 | — | 26,2 |
| 20 | — | 36,3 | 29 | — | 23,6 |
| 21 | — | 35,7 | 30 | — | 22,0 |
| 22 | — | 34,4 | 31 | — | 19,4 |
| 23 | — | 33,8 | 32 | — | 18,1 |
| 24 | — | 30,6 | 33 | — | 17,1 |
| 25 | — | 30,2 | 34 | — | 13,5 |
| 26 | — | 30,2 | 35 | — | 11,7 |
| 27 | — | 28,2 | | | |

Wirkstoff B

Massenspektrum

Durch Hochauflösung wird die Summenformel zu $C_{35}H_{59}NO_8$ ermittelt.

Gefunden wurde: 621.4257, für obige Summenformel berechnet: 621.4240 .

Das niederaufgelöste Massenspektrum zeigt die gleichen Fragmentierungen

wie für A und A-1: $M^+-CH_3OH - H_2O-H_2O-H_2O$.

UV-Spektrum

Lamda $_{max}$ = 238 nm (epsilon= 20.000), 236 nm (epsilon= 20.200), Methanol.
280 nm (epsilon = 300)

IR-Spektrum

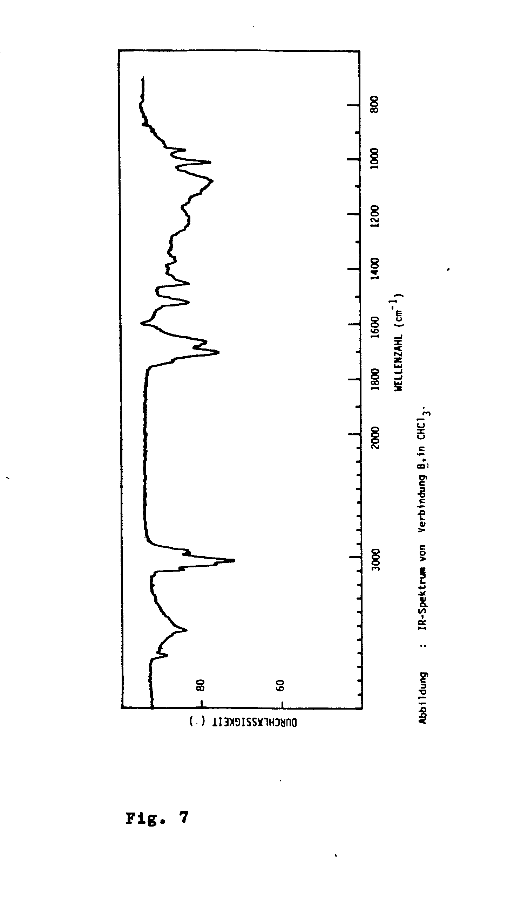

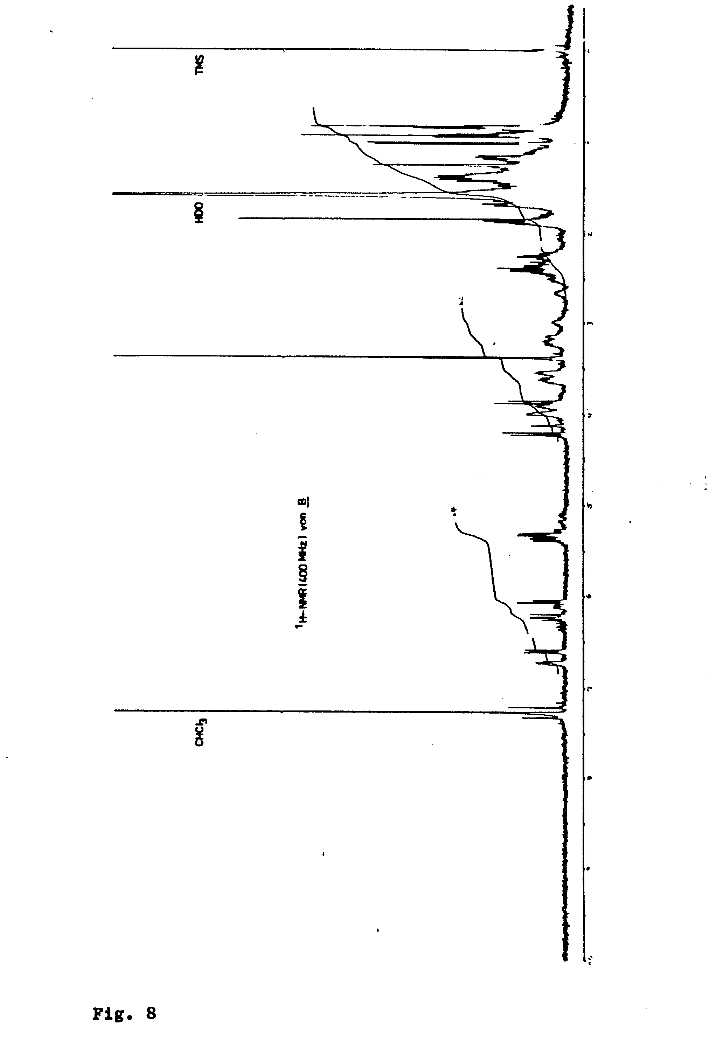

siehe Abbildung 7

$^1$H-NMR Spektrum

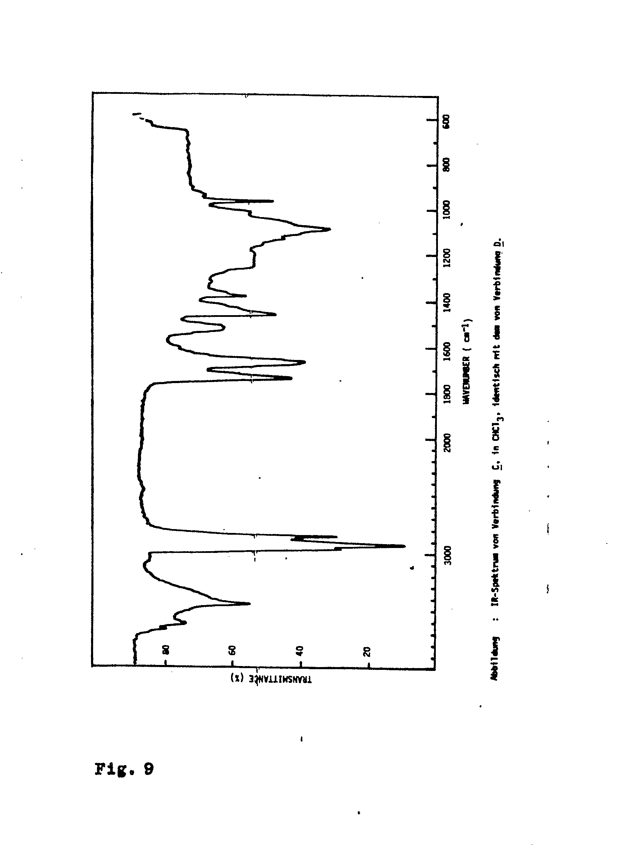

siehe Abbildung 8 Laufverhalten auf der Dünnschichtplatte (Kieselgel) im System Aceton:Hexan=1:1  :  $R_f$= 0.12 Wirkstoffe C und D

Massenspektrum

Die Summenformel $C_{35}H_{63}NO_7$ wird durch Hochauflösung ermittelt.

Gef.: 609.4599, für die Summenformel berechnet: 609,4604.

Die niederaufgelösten Massenspektren zeigen für beide Verbindungen ebenfalls die Fragmentierungen : $M^+-CH_3OH-H_2O-H_2O-H_2O$.

UV-Spektren

lamda$_{max}$= 238 nm (epsilon = 21000), keine Absorption bei 280 nm.


IR-Spektren

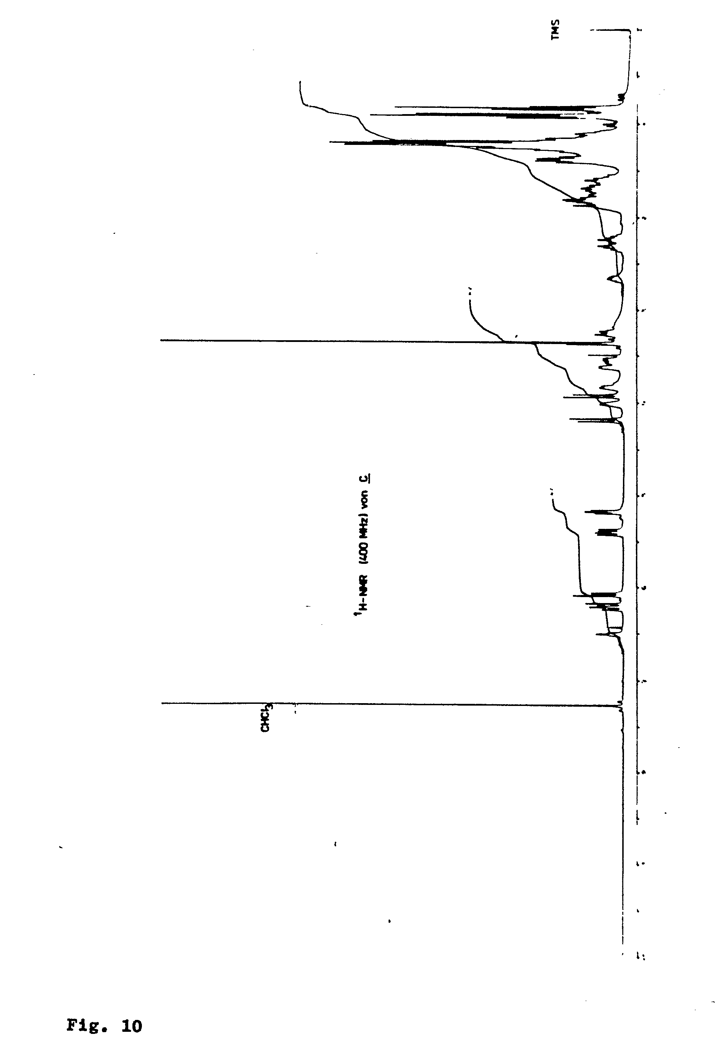

siehe Abbildung 9

Optische Drehungen

C: $\alpha_D$= + 22.2$^0$  (c=0.3 ,CHCl$_3$),

D: $\alpha_D$= + 6.3$^0$  (c=0.25,CHCl$_3$)

$^1$H-NMR Spektrum von C

siehe Abbildung 10

$^1$H-NMR Spektrum von D

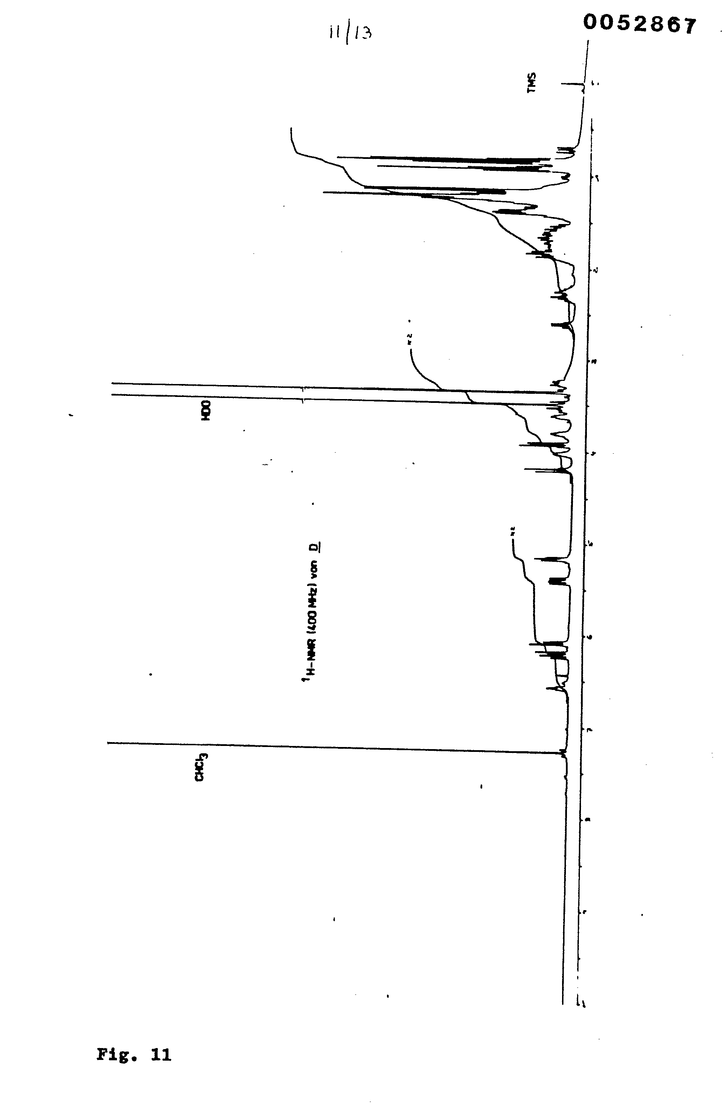

siehe Abbildung 11

**0052867**

Laufverhalten von C (Kieselgel)

Hexan:Aceton=1:1  : $R_f$= 0.18

Laufverhalten von D (Kieselgel)

Hexan:Aceton=1:1  : $R_f$= 0.21

Wirkstoff E

Für den Peak im Elutions-Diagramm (siehe Abbildung 1) bei der Retentions - zeit 3.04 sind .                   zwei Substanzen verantwortlich.

Die letzte von ihnen mit der angegebenen Zeit ließ sich präparativ abtrennen und zeigte die folgenden Eigenschaften.

Massenspektrum

Durch Hochauflösung wurde die Summenformel $C_{32}H_{55}NO_8$ bestimmt.

Gef.: 581.39200, für die Formel ber.: 581.39274.

Das niederaufgelöste Spektren erbrachte erneut die für A beschriebenen Fragmentierungen.

UV-Spektrum

lamda$_{max}$ (nm)= 238(epsilon=20.000), 232(epsilon= 21.000), Methanol.
280(epsilon=300)

IR-Spektrum

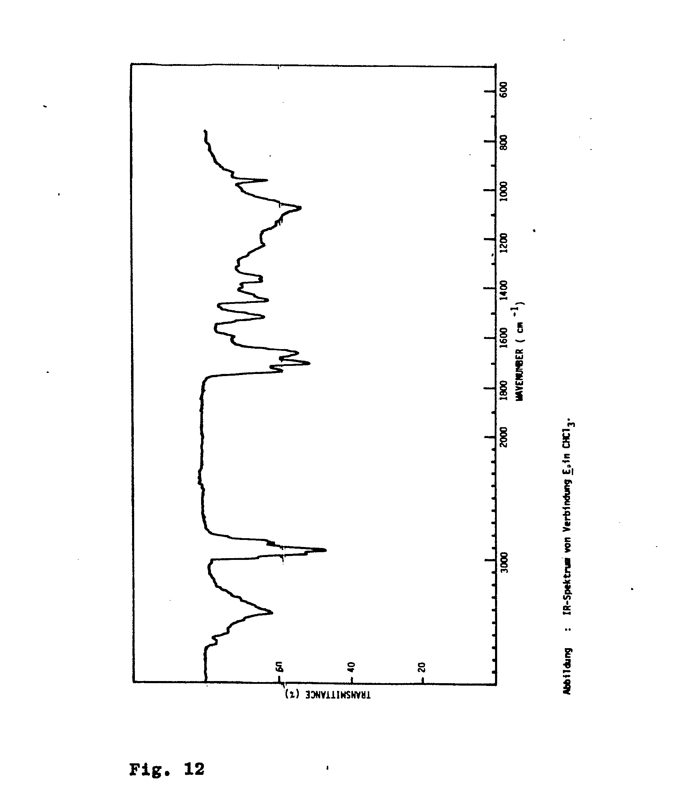

siehe Abbildung 12

$^1$H-NMR Spektrum

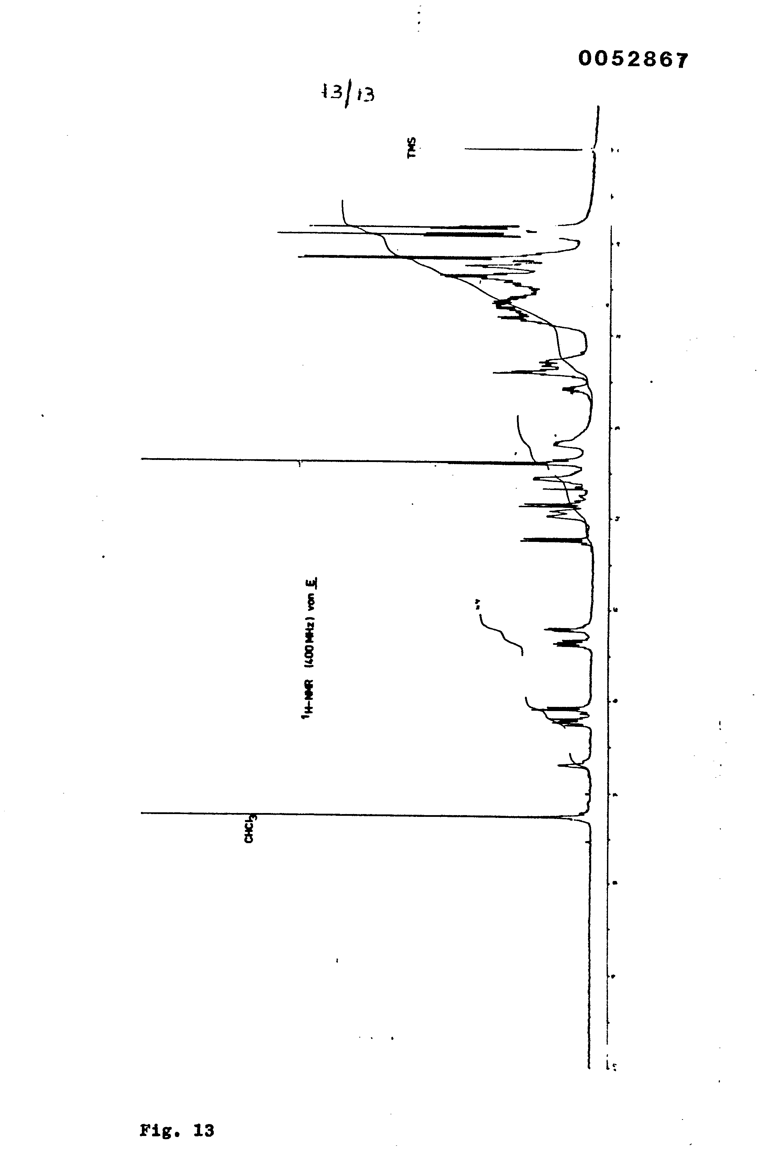

siehe Abbildung 13 Laufverhalten:  Hexan:Aceton=1:1  : $R_f$=0.10    (Kieselgel) Nachstehend wird die Erfindung durch Beispiele näher erläutert.

Fermentation im Fermenter

Ein typischer Fermentationsverlauf des Stammes Mx-v-48 M8 ist auf den Seiten 4-8 beschrieben und dargestellt.

Die Ausbeute an Antibiotikum bezogen auf die Hauptkomponente A betrug 2-2.5 mg im Durchschnitt. Die Fermentationen in 300 l- bzw. 700 l-Fermentern führte zu entsprechenden Ergebnissen.

Gewinnung des Wirkstoffgemisches aus dem Kulturüberstand

Zur Gewinnung der biologisch aktiven Substanzen wurden die Zellen vom Nährboden abzentrifugiert. Die Kulturbrühe wurde mittels einer

Gegenstromextraktion mit Essigester extrahiert . Alternativ wurde durch
Einrühren von XAD-2 oder XAD-4 in die abgetrennte Kulturbrühe, Elution 0052867
des von der Brühe abgetrennten XAD-4 oder XAD-2 mittels Aceton das Wirkstoffgemisch erhalten. Die jeweiligen Extrakte wurden entwässert, eingeengt
und zwischen Methanol und Hexan zur Abtrennung von unpolaren , nichtaktiven Substanzen verteilt. Die Methanol-Phase wurde eingeengt und über
Sephadex LH-20 chromatographiert.

Aus einem 70 l-Fermenter wurden aus etwa 30 g Methanol-Inhaltsstoffen
nach LH-20 Chromatographie etwa 600 mg eines biologisch-aktiven
Substanzgemisches erhalten.

Isolierung der einzelnen Wirkstoffe

Zur weiteren Reinigung wurde das erhaltene Rohgemisch an einer präparati -
ven Säule ( Waters-LC) mit Methanol und 10% Wasser als Laufmittel chromato -
graphiert. Die Auftrennung in die einzelnen Fraktionen gelang unter
Zuhilfenahme einer halbpräparativen Knauer-Säule (RP-18) . Eluiert
wurde mit einem Laufmittelgradienten, der in der Abbildung 1 angegeben ist.


Für die einzelnen Wirkstoffe von Mx-v-48 M8 zeigen :

für A und A-1:

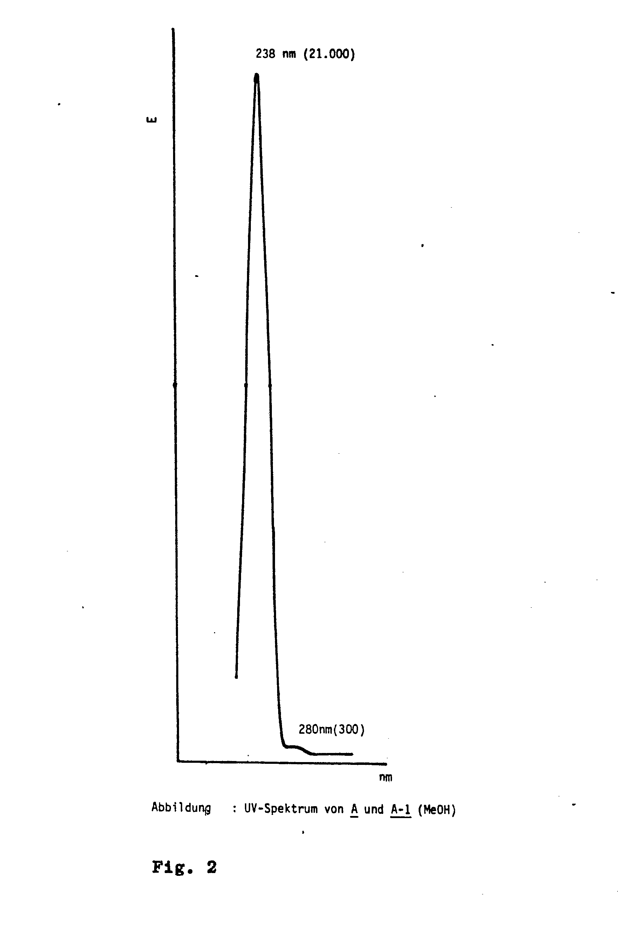

Abbildung 2 ein UV-Spektrum,

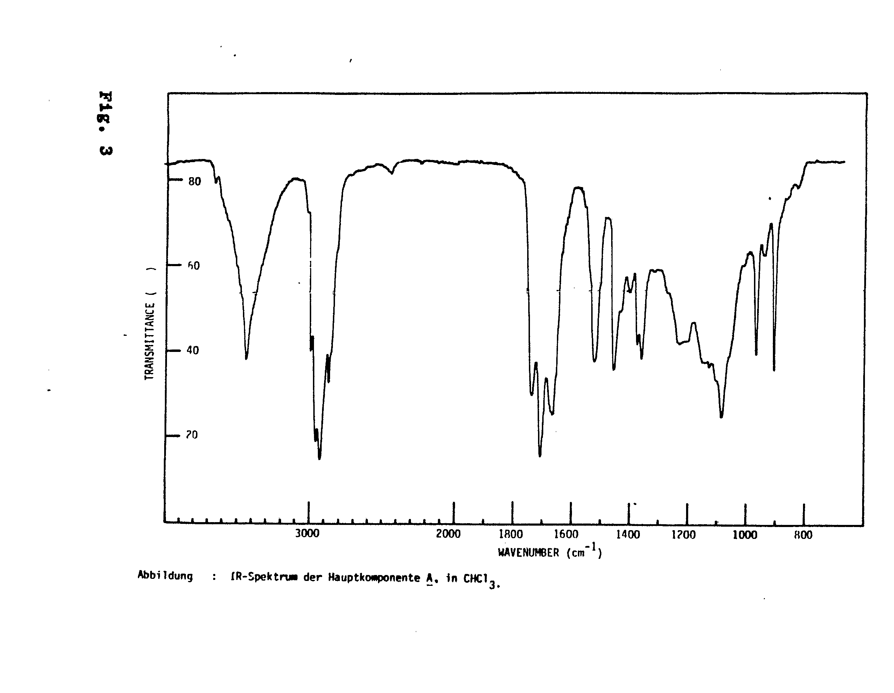

Abbildung 3 ein IR-Spektrum,

Abbildungen 4 und 5 je ein $^1$H-NMR Spektrum für A und A-1,

Abbildung 6 ein $^{13}$C-NMR Spektrum,

für Wirkstoff B :

Abbildung 7 ein IR-Spektrum,

Abbildung 8 ein $^1$H-NMR Spektrum,

für C und D :

Abbildung 9 ein IR-Spektrum,

Abbildung 10 ein $^1$H-NMR Spektrum für C und

Abbildung 11 ein $^1$H-NMR Spektrum für D,

für Wirkstoff E:

Abbildung 12 ein IR-Spektrum und

Abbildung 13 ein $^1$H-NMR Spektrum .

## Patentansprüche

1.   Kulturbrühe von Myxococcus virescens DSM 1888 oder DSM 2177, erhältlich durch Kultivieren von Myxococcus virescens DSM 1888 oder DSM 2177 in einem Kohlenstoff und Stickstoff sowie Mineralsalze enthaltenden wässerigen Nährmedium bei Temperaturen von etwa 15 bis 40 $^{o}$C.

2.   Kulturbrühe nach Anspruch 1, dadurch gekennzeichnet, daß sie durch Kultivieren von Myxococcus virescens DSM 1888 oder DSM 2177 bei 25 bis 35, insbesondere bei 30 $^{o}$C, erhältlich ist.

3.   Verfahren zur Herstellung einer Kulturbrühe gemäß Anspruch 1 oder 2, dadurch gekennzeichnet, daß man Myxococcus virescens DSM 1888 oder DSM 2177 in einem Kohlenstoff und Stickstoff sowie Mineralsalze enthaltenden wässerigen Medium bei etwa 15 bis 40 $^{o}$C kultiviert.

4.   Verfahren nach Anspruch 3, dadurch gekennzeichnet, daß man die Kultivierung bei 25 bis 35, insbesondere bei etwa 30 $^{o}$C, durchführt.

5.   Extrakt, erhältlich unter Verwendung der Kulturbrühe gemäß Anspruch 1 oder 2 durch Extrahieren der gesamten Kulturbrühe (ggf. nach einem Aufschluß der Zellen von Myxococcus virescens DSM 1888 oder DSM 2177), der von den Zellen von Myxococcus virescens DSM 1888 oder DSM 2177 abgetrennten Kulturbrühe oder der von der Kulturbrühe abgetrennten Zellen von Myxococcus virescens DSM 1888 oder DSM 2177 (ggf. nach einem Aufschluß der Zellen)
(a) mit einem organischen Lösungsmittel und Abtrennen der Extraktphase oder
(b) mit einem Adsorberharz, z.B. auf Polystyrolbasis, Eluieren des Adsorberharzes mit einem organischen Lösungsmittel und Abtrennen der Extraktphase.

6.    Extrakt nach Anspruch 5, dadurch gekennzeichnet, daß er gemäß (a) durch Extrahieren mit Essigsäureäthylester oder gemäß (b) durch Extrahieren mit XAD-4 oder XAD-2 und Eluieren mit Aceton erhältlich ist.

7.    Verfahren zur Herstellung eines Extraktes gemäß einem der Ansprüche 5 und 6, dadurch gekennzeichnet, daß man das Verfahren gemäß Abschnitt (a) oder (b) von Anspruch 5 durchführt.

8.    Verfahren nach Anspruch 7, dadurch gekennzeichnet, daß man gemäß Anspruch 5 (a) mit Essigsäureäthylester extrahiert oder gemäß Anspruch 5 (b) mit XAD-4 oder XAD-2 extrahiert und mit Aceton eluiert.

9.    Wirkstoffgemisch, erhältlich aus dem Extrakt gemäß einem der Ansprüche 5 und 6 durch Verteilung des Extraktes zwischen einer polaren und einer unpolaren organischen Phase, Chromatographieren der eingeengten polaren organischen Phase und nachfolgendes Einengen des Eluats.

10.    Wirkstoffgemisch nach Anspruch 9, dadurch gekennzeichnet, daß es aus dem Extrakt gemäß Anspruch 5 oder 6 durch
a) Abdestillieren des organischen Lösungsmittels,
b) Trocknung (vor oder nach Stufe a),
c) Verteilung des Rückstandes zwischen einer polaren und einer unpolaren organischen Phase,
d) Einengen der polaren organischen Phase,
e) Chromatographieren der eingeengten polaren organischen Phase über Sephadex LH-20 und
f) Einengen der Laufmittelphase
erhältlich ist.

11. Wirkstoffgemisch nach Anspruch 10, dadurch gekennzeichnet, daß es durch Abdestillieren bzw. Einengen unter reduziertem Druck erhältlich ist.

12. Verfahren zur Herstellung eines Wirkstoffgemisches gemäß Anspruch 9, 10 oder 11, dadurch gekennzeichnet, daß man die Maßnahmen gemäß Anspruch 9 durchführt.

13. Verfahren nach Anspruch 12, dadurch gekennzeichnet, daß man den Extrakt gemäß Anspruch 5 oder 6

a) durch Abdestillieren vom organischen Lösungsmittel befreit,

b) den erhaltenen Rückstand trocknet (oder den Extrakt vor Stufe a) trocknet),

c) den Rückstand zwischen einer polaren und einer unpolaren organischen Phase verteilt,

d) die polare organische Phase einengt,

e) die eingeengte polare organische Phase über Sephadex LH-20 chromatographiert und

f) die Laufmittelphase einengt.

14. Verfahren nach Anspruch 13, dadurch gekennzeichnet, daß man das Abdestillieren bzw. Einengen unter reduziertem Druck durchführt.

15. Fester Wirkstoff, gekennzeichnet durch folgende Parameter:

ein triklines Kristallgitter $\underline{P}1$ mit den Gitterkonstanten

$\underline{a}$ = 14.412 (6), $\underline{b}$ = 22.650 (6), $\underline{c}$ = 6.238 (3) $Å$,

alpha = 90.00 (2), ß = 102.56 (4), gamma = 90.04 (3) $^{\circ}$,

$\underline{z}$ = 2, $\underline{D}_{ber}$ = 1.04 g . $cm^3$;

einen FP. von 42 bis 43 $^{\circ}$C;

Nadeln aus Dichlormethan/n-Hexan-Gemischen;

UV-Löschung auf Kieselgel $F_{254}$;

den $R_f$-Werten bei Chromatographie auf Kieselgel mit Essigsäureäthylester 0,1, mit Dichlormethan/Methanol (15/1)

- 4 -

0,43, mit Hexan/Isopropanol (4/1) 0,17 und mit Hexan/Aceton
(1/1) 0,14,

eine optische Drehung alpha$_D$ = + 27,3 $^O$, c = 1 (Methanol);
und die charakteristischen Spektren:

UV in Methanol (Figur 2) mit einem Wert von lambda$_{max}$ von
238 bei einem Extinktionskoeffizienten von 21 800 und einer
Schulter bei 280 nm bei einem Extinktionskoeffizienten von
300;

ein Massenspektrum mit einem Molekülion-Peak für $C_{35}H_{61}NO_8$
und Fragmentionen-Peaks für $C_{34}H_{57}NO_7$ und $C_{29}H_{49}NO_5$;

ein IR-Spektrum in $CHCl_3$ (Figur 3) mit den Werten für $\overline{\gamma}$=
3450, 3000, 2970, 2930, 2875, 1740, 1707, 1665, 1525, 1460,
1400, 1380, 1360, 1240, 1090, 970 und 908 cm$^{-1}$;

ein $^1$H-NMR-Spektrum in $CDCl_3$ gemäß Figur 5;

ein $^{13}$C-NMR-Spektrum in $CDCl_3$ gemäß Figur 6 mit den chemischen Verschiebungen (delta, ppm) der entsprechend numerierten C-Atome (Multiplizitäten im Off-Resonanz-Spektrum:
s = Singulett, d = Dublett, t = Triplett, q = Quartett):

| Nr. | | δ | Mult. | Gruppe | | Nr. | | δ | Mult. | Gruppe |
|---|---|---|---|---|---|---|---|---|---|---|
| 1 | - | 212,2 | s | C-CO-C | | 19 | - | 37,0 | d | -CH< |
| 2 | - | 176,1 | s | C-CO-O | | 20 | - | 36,3 | t | |
| 3 | - | 171,1 | s | C-CO-NH | | 21 | - | 35,7 | t | |
| 4 | - | 139,2 | d | =CH- | | 22 | - | 34,4 | t | |
| 5 | - | 134,9 | s | =C< | | 23 | - | 33,8 | t | |
| 6 | - | 129,3 | d | =CH- | | 24 | - | 30,6 | t | |
| 7 | - | 125,7 | d | =CH- | | 25 | - | 30,2 | d | -CH< |
| 8 | - | 74,0 | d | >CH-O | | 26 | - | 30,2 | t | |
| 9 | - | 73,5 | d | >CH-O | | 27 | - | 28,2 | t | |
| 10 | - | 71,2 | d | >CH-O | | 28 | - | 26,2 | t | |
| 11 | - | 70,5 | t | -CH$_2$-OCH$_3$ | | 29 | - | 23,6 | t | |
| 12 | - | 67,7 | d | >CH-O | | 30 | - | 22,0 | t | |
| 13 | - | 57,7 | q | O-CH$_3$ | | 31 | - | 19,4 | q | CH$_3$ |
| 14 | - | 45,3 | t | | | 32 | - | 18,1 | t | |
| 15 | - | 44,9 | d | -CH< | | 33 | - | 17,1 | q | CH$_3$ |
| 16 | - | 43,0 | t | | | 34 | - | 13,5 | q | CH$_3$ |
| 17 | - | 42,1 | t | | | 35 | - | 11,7 | q | CH$_3$ |
| 18 | - | 40,6 | t | | | | | | | |

und durch Abtrennbarkeit aus einem Wirkstoffgemisch gemäß
Anspruch 9, 10 oder 11,

und sein Isomeres.


16.    Fester Wirkstoff, gekennzeichnet durch folgende Parameter:

den $R_f$-Wert bei Chromatographie auf Kieselgel mit Aceton/
Hexan (1/1) 0,12; und

die charakteristischen Spektren:

UV in Methanol mit einem Wert von $lambda_{max}$ von 280 bei
einem Extinktionskoeffizienten von 300,
238 bei einem Extinktionskoeffizienten von 20.000 und
236 nm bei einem Extinktionskoeffizienten von 20.200;

einem Massenspektrum entsprechend der Summenformel $C_{35}H_{59}NO_8$;

einem IR-Spektrum gemäß Figur 7; und

einem $^1$H-NMR-Spektrum gemäß Figur 8; und
durch Abtrennbarkeit aus einem Wirkstoffgemisch gemäß Anspruch 9, 10 oder 11.


17.    Fester Wirkstoff, gekennzeichnet durch folgende Parameter:

den $R_f$-Wert bei Chromatographie auf Kieselgel mit Hexan/
Aceton (1/1) 0,18;

eine optische Drehung $alpha_D$ = + 22,2 $^o$, c = 0,3 (Chloroform); und

die charakteristischen Spektren:
UV mit einem Wert von $lambda_{max}$ von 238 nm bei einem Extinktionskoeffizienten von 21.000 (keine Absorption bei 280 nm);

einem Massenspektrum entsprechend der Summenformel
$C_{35}H_{63}NO_7$;
einem IR-Spektrum gemäß Figur 9; und

einem $^1$H-NMR-Spektrum gemäß Figur 10; und

durch Abtrennbarkeit aus einem Wirkstoffgemisch gemäß Anspruch 9, 10 oder 11.

18.    Fester Wirkstoff, gekennzeichnet durch folgende Parameter:

den $R_f$-Wert bei Chromatographie auf Kieselgel mit Hexan/ Aceton (1/1) 0,21;

eine optische Drehung $alpha_D$ = + 6,3 $^O$, c = 0,25 (Chloroform); und

die charakteristischen Spektren:

UV mit einem Wert von $lambda_{max}$ von 238 nm bei einem Extinktionskoeffizienten von 21.000 (keine Absorption bei 280 nm);

einem Massenspektrum entsprechend der Summenformel $C_{35}H_{63}NO_7$;

einem IR-Spektrum gemäß Figur 9; und

einem $^1$H-NMR-Spektrum gemäß Figur 11; und

durch Abtrennbarkeit aus einem Wirkstoffgemisch gemäß Anspruch 9, 10 oder 11.

19.    Fester Wirkstoff, gekennzeichnet durch folgende Parameter:

den $R_f$-Wert bei Chromatographie auf Kieselgel mit Hexan/ Aceton (1/1) 0,10; und

die charakteristischen Spektren:

UV in Methanol mit einem Wert von $lambda_{max}$ von 280 bei einem Extinktionskoeffizienten von 300,
238 bei einem Extinktionskoeffizienten von 20.000 und
232 nm bei einem Extinktionskoeffizienten von 21.000;

einem Massenspektrum entsprechend der Summenformel $C_{32}H_{55}NO_8$;

einem IR-Spektrum gemäß Figur 12; und

einem $^1$H-NMR-Spektrum gemäß Figur 13; und

durch Abtrennbarkeit aus einem Wirkstoffgemisch gemäß Anspruch 9, 10 oder 11.

20. Wirkstoffe, gekennzeichnet durch folgende Parameter:

| Wirkstoff | Retentionszeit (min) |
|-----------|----------------------|
| 1 | ca. 2,9 |
| 2 | 6,79 |
| 3 | 10,89 |
| 4 | 11,65 |
| 5 | 15,95 |

im HPLC-Elutionsdiagramm gemäß Figur 1 (RP-18; 7 /um;
Gradient:
A (Methanol : Wasser = 85/15), B (Methanol/Wasser = 90/10);
unterbrochene Linie : B (%) = 0 bis 100 %; Fluß : 2 ml/min;
Detektor : 254 nm)
für folgende Standards:

| Wirkstoff gemäß Anspruch | Retentionszeit (min) |
|--------------------------|----------------------|
| 19 | 3,04 |
| 16 | 4,09 |
| 15 (Isomerengemisch) | 4,52 |
| 17 | 16,62 |
| 18 | 17,12 |

und durch Abtrennbarkeit aus einem Wirkstoffgemisch gemäß
Anspruch 9, 10 oder 11.

21. Verfahren zur Herstellung eines Wirkstoffes gemäß einem
der Ansprüche 15 bis 20, dadurch gekennzeichnet, daß man
das Wirkstoffgemisch gemäß einem der Ansprüche 9, 10 oder
11 an einer mit reversed-phase-Kieselgel beschickten Säule
mit einem polaren organischen Lösungsmittel unter Zusatz von
Wasser chromatographiert, den Wirkstoff als letzte Fraktion
abtrennt, durch Abdampfen des Laufmittels vor oder nach dem
Trocknen und ggf. fraktionierte Kristallisation isoliert.

22.    Mikroorganismus DSM 1888.

22.    Mikroorganismus DSM 2177.

HPLC-Elutions-Diagramm der Komponenten mit biologischer Wirkung
von Mx-v-48. Material: RP-18, 7 μ; Gradient: A(Methanol:Wasser=85/15),
B(Methanol:Wasser = 90/10), unterbrochene Linie: %B (0-100%);
Fluß: 2 ml/min; Detektor : 254 nm.
Alle Verbindungen, ausgenommen die bei $R_T$= 1.79 min, sind biologisch
aktiv.

**Fig. 1**

238 nm (21.000)

E

280nm(300)

nm

Abbildung : UV-Spektrum von A und A-1 (MeOH)

Fig. 2

Abbildung : IR-Spektrum der Hauptkomponente A, in CHCl₃.

Fig. 3

Fig. 4

4/13

CHCl₃    ¹H-NMR (400 MHz) von A

0052867

5/13

$^1$H-NMR (400 MHz) von A-1

Fig. 5

Fig. 6: $^{13}$C-Spektrum von A-1

Fig. 7

DURCHLÄSSIGKEIT (·)

80

60

3000    2000    1800    1600    1400    1200    1000    800

WELLENZAHL (cm$^{-1}$)

Abbildung    :    IR-Spektrum von  Verbindung B, in CHCl$_3$.

Fig. 8

$^1$H-NMR (400 MHz) von **B**

CHCl$_3$

HDO

TMS

8/13

0052867

Abbildung : IR-Spektrum von Verbindung C. in CHCl₃, identisch mit dem von Verbindung D.

Fig. 9

Fig. 10

CHCl₃

¹H-NMR (400 MHz) von C

TMS

Fig. 11

$^1$H-NMR (400 MHz) von D

TMS

HDO

CHCl$_3$

Fig. 12

$^1$H-NMR (400 MHz) von E

Fig. 13

0052867

# EUROPÄISCHER RECHERCHENBERICHT

Nummer der Anmeldung

EP 81 10 9783

Europäisches
Patentamt

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe. soweit erforderlich. der maßgeblichen Teile | betrifft Anspruch | |
| A | T. KORZYBSKI et al.: "Antibiotics", Band 1, Pergamon Press, Seite 159 PWN-Polish Scientific Publishers "Antibiotic substances from myxococcaceae" * Insgesamt .* | 1,4 | C 12 P 1/04 C 12 N 1/20// (C 12 P 1/04 C 12 R 1/01) |
| P | EP - A - 0 022 425 (CIBA, GBF) * Insgesamt * | 1,4 | |
| A | CHEMICAL ABSTRACTS, Band 75, Nr. 17, 1971, Seite 30, Nr. 105335q Columbus, Ohio; U.S.A. G. HASKA: "Extracellular lytic enzymes of Myxococcus virescens. I. Separation of the bacteriolytic enzymes from the bulk of the proteinases" & PHYSIOL. PLANT. 1971, 25(1), 85-89 * Zusammenfassung * | 1 | **RECHERCHIERTE SACHGEBIETE (Int Cl.³)** C 12 P 1/00 C 12 N 1/00 C 12 R 1/00 |

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument. das jedoch erst am oder nach dem Anmeldedatum veröffentlicht worden ist
D: in der Anmeldung angefuhrtes Dokument
L: aus andern Grunden angeführtes Dokument
&: Mitglied der gleichen Patentfamilie, übereinstimmendes Dokument

Der vorliegende Recherchenbericht wurde fur alle Patentansprüche erstellt.

| Recherchenort | Abschlußdatum der Recherche | Prüfer |
|---|---|---|
| Den Haag | 12-02-1982 | RAJIC |

EPA form 1503.1 06.78